(19) Europäisches Patentamt
European Patent Office
Office européen des brevets

(11) Veröffentlichungsnummer: **0 147 639**
**B1**

# (12) EUROPÄISCHE PATENTSCHRIFT

(45) Veröffentlichungstag der Patentschrift: **22.07.87**

(51) Int. Cl.⁴: **C 01 B 21/14**, C 07 C 51/42

(21) Anmeldenummer: **84114250.8**

(22) Anmeldetag: **26.11.84**

(54) **Stabilisierte Lösungen von Hydroxylamin oder dessen Salze.**

(30) Priorität: **02.12.83 DE 3343600**

(43) Veröffentlichungstag der Anmeldung: **10.07.85 Patentblatt 85/28**

(45) Bekanntmachung des Hinweises auf die Patenterteilung: **22.07.87 Patentblatt 87/30**

(84) Benannte Vertragsstaaten: **BE CH DE FR GB IT LI NL**

(56) Entgegenhaltungen:
**US-A-3 145 082**
**US-A-3 480 391**
**US-A-3 480 392**
**US-A-3 544 270**

(73) Patentinhaber: **BASF Aktiengesellschaft, Carl-Bosch- Strasse 38, D-6700 Ludwigshafen (DE)**

(72) Erfinder: **Grosskinsky, Otto- Alfred, Dr.- Chem., Semmelweisstrasse 8, D-6700 Ludwigshafen (DE)**
Erfinder: **Frommer, Elmar, Dr.- Chem., Lisztstrasse 117, D-6700 Ludwigshafen (DE)**
Erfinder: **Ritz, Josef, Dr.- Chem., Osloer Weg 8, D-6700 Ludwigshafen (DE)**
Erfinder: **Thomas, Erwin, Borngasse 12, D-6713 Freinsheim (DE)**
Erfinder: **Weiss, Franz- Josef,Dr.- Chem., Schilfweg 1, D-6706 Neuhofen (DE)**

EP 0 147 639 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylanin und dessen Salzen zu stabilisieren, um eine verbesserte Lagerfähigkeit zu erzielen. So verwendet man als Stabilisierungsmittel Harnstoffderivate entsprechend der US-A-3 544 270. In der US-A-3 480 391 werden Amidoxime als geeignete Stabilisatoren empfohlen. Auch Hydroxamsäuren eignen sich entsprechend der US-A-3 480 392 zur Stabilisierung von Hydroxylamin. Aus der US-A-3 145 082 ist auch schon bekannt, chelatbildende Mittel, wie das Natriumsalz der Ethylendiamintetraessigsäure als Stabilisierungsmittel zu verwenden. Die bislang verwendeten Stabilisierungsmittel sind noch verbesserungsbedürftig.

Es war die technische Aufgabe gestellt, stabilisierte Lösungen von Hydroxylamin oder dessen Salze zur Verfügung zu stellen, die über einen längerem Zeitraum stabil sind und bei denen insbesondere die Zersetzung von freien Hydroxylamin minimiert wird.

Diese Aufgabe wird gelöst durch stabilisierte Lösungen von Hydroxylamin oder dessen Salze in Wasser oder Alkoholen, gekennzeichnet durch einen Gehalt an Hydroxyanthrachinonen.

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelöst enthaltenen molekularen Sauerstoff durch Behandeln mit Stickstoff, der frei von molekularem Sauerstoff ist, entfernt und dann Hydroxyanthrachinone zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auf ein Minimum reduziert wird.

Erfindungsgemäß geht man von Lösungen von Hydroxylamin oder dessen Salzen in Wasser oder Alkohole,, z.B. $C_1$- bis $C_4$-Alkanolen aus. Geeignete Salze des Hydroxylamins sind beispielsweise solche mit starken Mineralsäuren, wie Schwefelsäure, Salpetersäure, Chlorwasserstoffsäure oder solche mit Fettsäuren, z.B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salzen beträgt in der Regel von 10 bis 70 Gew.-%. In der Regel haben solche Lösungen einen pH-Wert von 8 bis 11. Besonders bevorzugt geht man von wäßrigen Lösungen aus.

Als Stabilisatoren verwendet man Hydroxyanthrachinone, insbesondere solche, die mindestens zwei Hydroxylgruppen enthalten. Bevorzugte Hydroxyanthrachinone sind solche der Formel

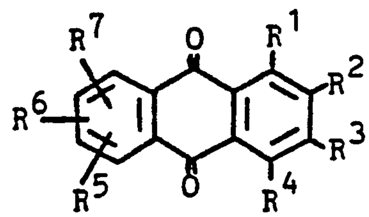

I,

im der $R^1$ bis $R^7$ jeweils für ein Wasserstoffatom, eine Hydroxylgruppe, eine $C_1$- bis $C_4$-Alkoxygruppe stehen, $R^3$ auch eine Methylgruppe bezeichnen kann, mit der Maßgabe, daß mindestens zwei Substituenten eine Hydroxylgruppe bedeuten. In besonders bevorzugten Hydroxyanthrachinonen der Formel 1 bezeichnen die Reste $R^1$ bis $R^7$ jeweils ein Wasserstoffatom, eine Hydroxylgruppe oder eine Methoxygruppe, mit der Maßgabe, daß mindestens 2 Substituenten eine Hydroxylgruppe bedeuten. Die Hydroxylgruppen enthaltenden Hydroxyanthrachinone können auch als Glykoside vorliegen. Geeignete Hydroxyantrhachinone sind beispielsweise 1,2-Dihydroxyanthrachinon, 1,4-Dihydroxyanthrachinon, 1,2,4-Trihydroxyanthrachinon, 2,3-Dihydroxyanthrachinon, 1,5-Dihydroxyanthrachinon, 1,8-Dihydroxyanthrachinon, 1,2,6-Trihodryxyanthrachinon, 1,2,7-Trihydroxyanthrachinon, 1,2,5,8-Tetrahydroxyanthrachinon, 1,2,4,5,8-Pentahydroxyanthrachinon, 1,6,8-Trihydroxy-3-methylanthrachinon, 1,8-Dioxy-3-methylanthrachinon, 1,8-Dihydroxy-3-methyl-6-methoxyanthrachinon. Vorteilhaft wendet man Hydroxyanthrachinone in Mengen von 0,005 bis 1 Gew.-%, insbesondere von 0,01 bis 0,1 Gew.-%, bezogen auf die zu stabilisierende Lösung an. Es hat sich ferner bewährt, wenn man zusätzlich Polyhydroxybenzole, insbesondere Pyrogallol mitverwendet. Polyhydroxybenzole werden vorteilhaft in Mengen von 0,005 bis 0,1 Gew.-%, bezogen auf die zu stabilisierende Lösung zugesetzt. Es ist bemerkenswert, daß durch die kombinierte Anwendung von Hydroxyanthrachinonen und Polyhydroxybenzolen eine synergistische Wirkung erzielt wird.

Stabilisierte Lösungen von Hydroxylamin oder dessen Salzen in Wasser oder Alkoholen werden erfindungsgemäß hergestellt, indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch, daß man durch die zu stabilisierende Lösung sauerstoffreien Stickstoff leitet, z.B. für 5 bis 10 Minuten. Der hierfür verwendete Stickstoff hat vorteilhaft einen Sauerstoffgehalt von kleiner 2 ppm. Anschließend gibt man Hydroxyanthrachinone und gegebenenfalls Polyhydroxybenzole zu und löst diese in der zu stabilisierenden Lösung auf. Es ist auch möglich, die

Stabilisatoren bereits in gelöstem Zustand, z.B. gelöst in $C_1$- bis $C_4$-Alkanolen den zu stabilisierenden Lösungen zuzufügen. Vorteilhaft hält man hierbei eine Temperatur von 5 bis 40° C ein.

Es versteht sich, daß es von Vorteil ist, eine Kontamination der zu stabilisierenden Lösungen mit Schwermetallen, insbesondere Kupfer oder Edelmetallen zu vermeiden, da diese die Zersetzung von Hydroxylamin katalysieren. Ferner ist es von Vorteil, energiereiche Strahlen durch geeignet eingefärbte Glasbehälter auszuschalten. Schließlich ist es von Vorteil, die stabilisierten Lösungen bei Temperaturen von kleiner 40° C, z.B. bei Temperaturen von 5 bis 20° C zu lagern.

Stabilisierte Lösungen von Hydroxylamin oder deren Salze eignen sich zur Herstellung von Oximen.

Der erfindungsgemäße Gegenstand sei an folgendem Beispiel veranschaulicht.

**Beispiel**

Eine wäßrige Lösung von Hydroxylamin wird bei 20° C 10 Minuten mit sauerstoffreiem Stickstoff begast. Die Konzentration an Hydroxylamin, Art und Menge des Stabilisators sowie die in Abhängigkeit von Zeit und Temperatur erzielten Ergebnisse sind aus folgender Tabelle ersichtlich.

**Tabelle**

| Stabilisator | °C | | | | | |
|---|---|---|---|---|---|---|
| 50 ppm Chinalizarin | 5 | 0 | 480 | 622 | 1506 | Stunden |
| | | 141,37 | 141,32 | 141,22 | 140,61 | g/l $NH_2OH$ |
| " | | 0 | 480 | 619 | 1504 | Stunden |
| | 20 | 141,21 | 139,92 | 139,76 | 137,97 | g/l $NH_2OH$ |
| " | | 0 | 480 | 618 | 1486 | Stunden |
| | 40 | 141,05 | 139,20 | 138,60 | 135,99 | g/l $NH_2OH$ |

**Patentansprüche**

1. Stabilisierte Lösungen vom Hydroxylamin oder dessen Salzen in Wasser oder Alkoholen, gekennzeichnet durch einen Gehalt an Hydroxyanthrachinonen.

2. Stabilisierte Lösungen nach Anspruch 1, gekennzeichnet durch einen Gehalt an Hydroxyanthrachinonen der Formel

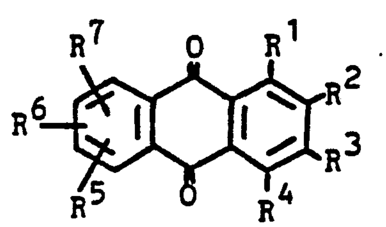

I,

in der $R^1$ bis $R^7$ jeweils für ein Wasserstoffatom, eine Hydroxylgruppe oder eine $C_1$- bis $C_4$-Alkoxygruppe stehen und $R^3$ auch einen Methylrest bezeichnen kann, mit der Maßgabe, daß mindestens zwei Substituenten eine Hydroxylgruppe bedeuten.

3. Stabilisierte Lösungen nach den Ansprüchen 1 und 2, gekennzeichnet durch einen Gehalt an Hydroxyanthrachinonen der Formel I, in der $R^1$ bis $R^7$ jeweils für ein Wasserstoffatom, eine Hydroxylgruppe oder eine Methoxygruppe stehen, $R^3$ auch einen Methylrest bezeichnen kann, mit der Maßgabe, daß mindestens zwei Substituenten eine Hydroxylgruppe bedeuten.

4. Stabilisierte Lösungen nach den Ansprüchen 1 bis 3, gekennzeichnet durch einen Gehalt von 0,005 bis 1 Gew.-% Hydroxyanthrachinonen, bezogen auf die zu stabilisierende Lösung.

5. Stabilisierte Lösungen nach den Ansprüchen 1 bis 4, gekennzeichnet durch einen zusätzlichen Gehalt an Pyrogallol.

6. Verfahren zur Herstellung von stabilisierten Lösungen von Hydroxylamin oder dessen Salze in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt und dann Hydroxyanthrachinone zusetzt.

**Claims**

1. A stabilized solution of hydroxylamine or its salts in water or an alcohol, which contains a hydroxyanthraquinone.

2. A stabilized solution as claimed in claim 1, which contains a hydroxyanthraquinone of the formula

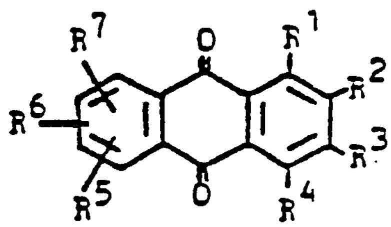

I,

where $R^1$, $R^2$, $R^3$, $R^4$, $R^5$, $R^6$ and $R^7$ are each hydrogen, hydroxyl or $C_1$-$C_4$-alkoxy, and $R^3$ may furthermore be methyl, with the proviso that at least two substitutents are hydroxyl.

3. A stabilized solution as claimed in claims 1 and 2, which contains a hydroxyanthraquinone of the formula I, where $R^1$, $R^2$, $R^3$, $R^4$, $R^5$, $R^6$ and $R^7$ are each hydrogen, hydroxyl or methoxy, and $R^3$ may furthermore be methyl, with the proviso that at least two substituents are hydroxyl.

4. A stibilized solution as claimed in claims 1 to 3, which contains from 0.005 to 1% by weight, based on the solution to be stabilized, of a hydroxyanthraquinone.

5. A stabilized solution as claimed in claims 1 to 4, which additionally contains pyrogallol.

6. A process for the preparation of a stabilized solution of hydroxylamine or its salts in water or an alcohol by adding a stabilizer, wherein the molecular oxygen dissolved in the solution to be stabilized is removed from this solution by treatment with nitrogen which is free of molecular oxygen, and a hydroxyanthraquinone is then added.

**Revendications**

1. Solutions stabilisées d'hydroxylamine ou ses sels dans l'eau ou les alcools, caractérisées par une teneur en hydroxyanthraquinone.

2. Solutions stabilisées selon la revendication 1, caractérisées par une teneur en hydroxyanthraquinone de formule

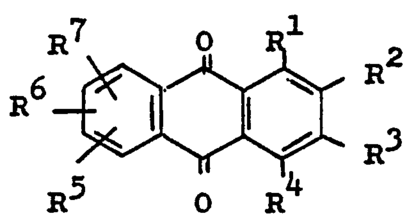

I,

dans laquelle $R^1$ à $R^7$ représentent chacun un atome d'hydrogène, un groupe hydroxyle ou un groupe alcoxy en $C_1$ à $C_4$ et $R^3$ peut aussi désigner un reste méthyle, sous réserve qu'au moins deux substituants représentent un groupe hydroxyle.

3. Solutions stabilisées selon les revendications 1 et 2, caractérisées par une teneur en hydroxyanthraquinone de formule I, dans laquelle $R^1$ à $R^7$ représentent chacun un atome d'hydrogène, un groupe hydroxyle ou un groupe méthoxy, $R^3$ peut aussi désigner un reste méthyle, sous réserve qu'au moins deux substituants représentent un groupe hydroxyle.

4. Solutions stabilisées selon les revendications 1 à 3, caractérisées par une teneur de 0,005 à 1 % en poids d'hydroxyanthraquinone, rapportée à la solution à stabiliser.

5. Solutions stabilisées selon les revendications 1 à 4, caractérisées par une teneur additionnelle en pyrogallol.

6. Procédé de préparation de solutions stabilisées d'hydroxylamine ou ses sels dans l'eau ou les alcools par

addition de stabilisants, caractérisé par le fait que l'on élimine des solutions à stabiliser l'oxygène moléculaire qui y est contenu dissous, par traitement avec de l'azote qui est exempt d'oxygène moléculaire, puis on ajoute de l'hydroxyanthraquinone.